# EUROPEAN PATENT APPLICATION

(11) **EP 4 368 127 A2**
(43) Date of publication of application: **15.05.2024**
(21) Application number: 23209138.9
(22) Date of filing: 10.11.2023
(51) Int. Cl.: A61B 17/17, A61B 17/72, A61B 17/80

(54) **SAGITTAL ADJUSTMENT SLOT IN PLATE USED WITH INTRAMEDULLARY NAIL**

(30) Priority: 10.11.2022 US 202263424296 P; 07.06.2023 US 202363471654 P
(71) Applicant: Stryker European Operations Limited, Carrigtwohill, Co. Cork T45 HX08 (IE)
(72) Inventor: ZANDER, Nils, 24340 Eckernförde (DE); WIELAND, Manfred, 24146 Kiel (DE); KLUEVER, Hendrik, 24232 Schoenkirchen (DE)
(74) Representative: Maiwald GmbH

(57) **Abstract**

A fracture fixation system (100) includes a bone nail (70) configured to be inserted into a canal of a bone, and a bone plate (10) configured to abut a surface of the bone, wherein the bone nail (70) includes first, second, and third nail holes (73, 74, 75), the second nail hole (74) located between the first and third nail holes (73, 75), and wherein the bone plate (10) includes first and third plate holes (14, 16) and a slot (15) located between the first and third plate holes (14, 16). In one embodiment, the slot (15) of the bone plate (10) has a center line (18) extending along an arc of a constant radius from a center point (17) of the first plate hole (14).

## Description

### CROSS-REFERENCE TO RELATED APPLICATIONS

The present application claims priority to U.S. Application No. 63/471,654 filed June 7, 2023, and to U.S. Application No. 63/424,296 filed November 10, 2022, the disclosures of which are incorporated herein by reference.

### BACKGROUND OF THE INVENTION

For the treatment of complex and often metaphyseal fractures, the combined utilization of both intramedullary nails and lateral or medial locking plates have been established as a viable clinical option. The resulting fixation strength and rigidity of this kind of combined osteosynthesis eventually allows for early or even immediate weight bearing protocols, thus positively influencing post-operative measures such as fracture healing, general patient outcome (quality of life) or even mortality rates.

An attempt to maximize biomechanical performance by combining both nails and plates is often enhanced by the in-situ generation of linked constructs by using screws and/or pegs passing into both the plate and intramedullary nail simultaneously. Although final clinical and mechanical evidence concerning the efficacy of such constructs is lacking, the current clinical literature assumes there is a positive impact on weight-bearing capabilities and fracture healing by providing additional stability and adapted construct stiffness.

Nevertheless, beside the unclear clinical efficacy of implant linking, the number of potential distal linking options remain controversial as well. While some publications refer to one (often the most distal nail locking hole) as sufficient, there may be a potential clinical benefit to provide additional distal linking options within a given Nail-Plate Combination (NPC) construct to enhanced intra-operative flexibility and stabilization potential.

Accordingly, further improvement in systems with interlocking intramedullary nails and locking plates is desired.

### BRIEF SUMMARY OF THE INVENTION

A first aspect of the present invention is a method of using a fracture fixation system, including inserting a bone nail into a canal of a bone; inserting a bone plate against a surface of the bone, locating a first end of the bone plate adjacent to a first end of the bone nail; inserting an alignment instrument through a first plate hole and through a first nail hole; aligning a third plate hole of the bone plate with a third nail hole of the bone nail; drilling a first bore in the bone through the first plate hole and through the first nail hole; inserting a first fastener into the first plate hole and the first nail hole to couple the bone nail and the bone plate; drilling a second bore in the bone through a slot in the bone plate and a second nail hole, the slot located between the first and third plate holes, and the second nail hole located between the first and third nail holes; and inserting a second fastener into the slot and the second nail hole.

In accordance with other embodiments of the first aspect, the method may further include inserting an alignment instrument through the third plate hole and the third nail hole. Each of the alignment instruments may be a k-wire. The step of inserting the bone nail may include inserting a retrograde femoral nail into an entry location at a distal end of a femur. The method may further include drilling a third bore through the third plate hole and the third nail hole, and inserting a third fastener into the third plate hole and the third nail hole. The step of inserting the first fastener, the step of inserting the second fastener, and/or the step of inserting the third fastener may include inserting a screw and threading a head of the screw into the first plate hole, the third plate hole, and/or the slot, respectively. The step of inserting the first fastener and the step of inserting the third fastener may include inserting a screw and threading a head of the screw into the first plate hole and the third plate hole, respectively, and the step of inserting the second fastener may include inserting a non-threaded head of the second fastener into the slot. Any of the steps of drilling may include using a targeting arm attached to the bone nail. Any of the steps of drilling may include using a drill sleeve. Any of the steps of drilling may include using a drill guide having a guide hole defined by a first conical surface and a second planar surface. Any of the steps of drilling may include using a drill guide having a guide hole defined only by a first conical surface and a second planar surface.

The method may further include drilling a fourth bore in the bone through a fourth plate hole of the bone plate and to avoid alignment with a hole in the bone nail, and inserting a fourth fastener into the fourth plate hole and avoiding contact of the fourth fastener with the bone nail. The method may further include drilling a fifth bore in the bone through a fifth plate hole of the bone plate and to avoid alignment with a hole in the bone nail, and inserting a fifth fastener into the fifth plate hole and avoiding contact of the fifth fastener with the bone nail. The step of drilling the fourth bore may include drilling the fourth bore to a first side of the bone nail, and the step of drilling the fifth bore may include drilling the fifth bore to a second side of the bone nail opposite the first side. The step of inserting the bone nail may include inserting the bone nail into a tibia. The step of inserting the bone nail may include inserting the bone nail into a humerus. The step of inserting the bone nail may include inserting the bone nail into a femur.

The first plate hole may be a slot. The steps of inserting the first fastener and inserting the second fastener may include inserting the first fastener and the second fastener into the first plate hole and the slot, respectively, without tightening the first fastener and the second fastener against the plate such that further movement of the plate with respect to the bone is permitted. After the steps of inserting the first fastener and inserting the second fastener, the method may further include moving the plate with respect to the bone in a direction that is substantially perpendicular to a longitudinal axis of the plate. The method may further include tightening the first fastener and the second fastener against the plate.

A second aspect of the present invention is a fracture fixation system, including a bone nail configured to be inserted into a canal of a bone, and a bone plate configured to abut a surface of the bone, wherein the bone nail includes first, second, and third nail holes, the second nail hole located between the first and third nail holes, and the bone plate includes first and third plate holes and a slot located between the first and third plate holes.

In accordance with other embodiments of the first aspect, a distance between a center point of the first plate hole to a center line of the slot may be substantially equal to a distance between a center point of the first nail hole to a center point of the second nail hole. The slot of the bone plate may have a center line extending along an arc of a constant radius from a center point of the first plate hole. A length of the slot along the centerline arc may be 3-25 mm.

The slot may extend substantially perpendicular to a midline of the bone plate extending between ends of the bone plate. The slot of the bone plate may have a center line extending along an arc of a constant radius, and the first plate hole may be a slot having a center line extending along an arc of a constant radius. The center points of the arcs may be the same or different. The slots may extend substantially perpendicular to a midline of the bone plate extending between ends of the bone plate. The slot of the bone plate may have a center line extending along an axis substantially perpendicular to a midline of the bone plate extending between ends of the bone plate.

A distance between a center point of the first plate hole to a center point of the third plate hole may be substantially equal to a distance between a center point of the first nail hole to a center point of the third nail hole. A perimeter of the slot may be scalloped to engage a head of a screw and to prevent backout.

The fracture fixation system may further include a first fastener disposed within the first plate hole and the first nail hole, a second fastener disposed within the third plate hole and the third nail hole, and a third fastener disposed within the slot and the second nail hole. At least one of the first, second, and third fasteners may be a screw. At least one of the first, second, and third fasteners may be an unthreaded peg. Each of the first plate hole, the third plate hole, the first nail hole, the second nail hole, and the third nail hole may have a circular perimeter, and the slot may have a non-circular perimeter. Each of the third plate hole, the first nail hole, the second nail hole, and the third nail hole may have a circular perimeter, the slot may have a non-circular perimeter, and the first plate hole may be a slot having a non-circular perimeter. The bone plate may further define a fourth plate hole located between a first side edge of the bone plate and a midline of the bone plate extending between ends of the bone plate. The first plate hole may define a perimeter confining a fastener to insertion along a single axis, and the fourth plate hole may define a perimeter confining a fastener to insertion along an axis parallel to the single axis. The bone plate may further define a fifth plate hole located between the midline of the bone plate and a second side edge of the bone plate that is opposite the first side edge. The fourth plate hole and the fifth plate hole may both intersect with a single axis that is perpendicular to the midline of the bone plate.

### BRIEF DESCRIPTION OF THE DRAWINGS

The drawings described herein are for illustrative purposes only of the selected embodiments and are not all possible implementations and thus are not intended to limit the scope of the present disclosure.
FIG. 1 illustrates a fracture fixation system in accordance with an embodiment of the present invention.
FIGS. 2 and 3 illustrate a bone plate of the fracture fixation system shown in FIG. 1.
FIGS. 4 and 5 illustrate a distal end of the bone plate of the fracture fixation system shown in FIG. 1.
FIGS. 6 and 7 illustrate perimeters of holes and a slot, respectively, in the bone plate of the fracture fixation system shown in FIG. 1
FIG. 8 illustrates a distal end of the bone plate of the fracture fixation system shown in FIG. 1.
FIG. 9 illustrates the fracture fixation system shown in FIG. 1 attached to a bone.
FIGS. 10-16 illustrate the use of a target guide in connection with the fracture fixation system shown in FIG. 1.
FIGS. 17-19 illustrate steps in a method of using the fracture fixation system shown in FIG. 1.
FIG. 20 illustrates a distal end of a bone plate in accordance with another embodiment of the present invention.
FIG. 21A illustrates the distal end of the bone plate shown in FIG. 20.
FIG. 21B illustrates an arrangement of slots in the bone plate shown in FIG. 21A.
FIG. 22A illustrates a distal end of a bone plate in accordance with another embodiment of the present invention.
FIG. 22B illustrates an arrangement of slots in the bone plate shown in FIG. 22A.
FIGS. 23A-D illustrate further arrangements of slots in a bone plate in accordance with other embodiments of the present invention.

### DETAILED DESCRIPTION

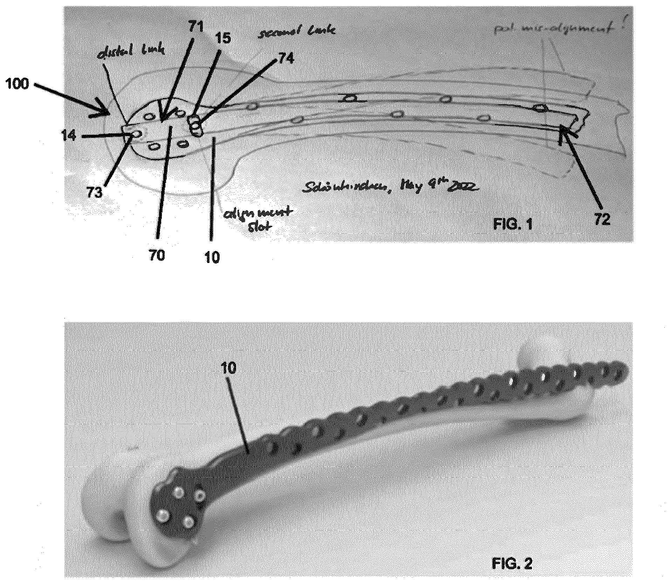

As shown in FIGS. 1-9, a fracture fixation system 100 in accordance with a first embodiment of the present invention includes a bone nail 70, a bone plate 10, and one or more fasteners, as described below. Nail 70 is an intramedullary nail for insertion into a canal of a long bone, such as a femur, humerus, tibia, fibula, etc. In one embodiment, nail 70 is a retrograde femoral nail. The geometry and relative dimensions of nail 70 can be designed for use with any particular long bone in which system 100 is intended to be used.

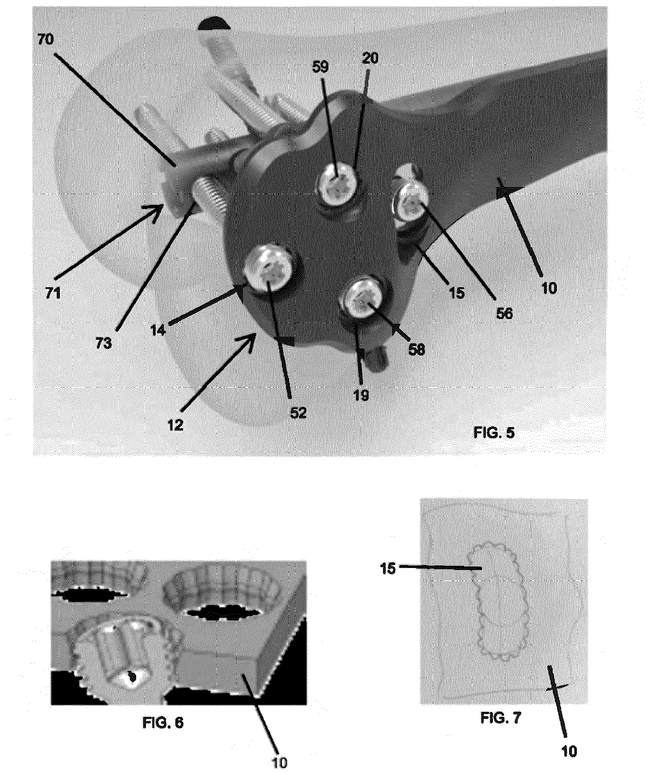

Nail 70 is generally longitudinal in shape and extends from a distal end 71 to a proximal end 72. Adjacent distal end 71, first and second distal nail holes 73, 74 are defined within nail 70, as shown in FIGS. 1 and 5. For the sake of referencing the configuration and use of nail 70 herein, first distal nail hole 73 is provided relatively nearer distal end 71, and second distal nail hole 74 is relatively closer to proximal end 72 of nail 70. At proximal end 72 of nail 70, a proximal nail hole 75 is defined within nail 70 and is proximally positioned with respect to both first and second distal nail holes 73, 74. Additional holes in any portion of nail 70 can be provided as may be suitable for purposes of additional fixation.

Plate 10 is also generally longitudinal in nature, and has an outer surface 11 and an inner surface configured to abut an outer surface of a bone. Plate 10 extends from a distal end 12 to a proximal end 13, such that distal end 12 of plate 10 is configured to be adjacent to distal end 71 of nail 70 for connection therewith when system 100 is assembled.

Adjacent distal end 12 of plate 10, a distal slot 15 and plate holes, herein referred to as a first distal plate hole 14, a fourth plate hole 19, and a fifth plate hole 20, are defined within plate 10. Similarly, and located proximally to first distal plate hole 14 and distal slot 15, a proximal plate hole 16 is defined adjacent proximal end 13 of plate 10. Plate holes 19 and 20 are each located between respective one of the opposing side edges of plate 10 and a midline of plate 10, the midline being a line that extends between distal and proximal ends 12, 13 of plate 10. In this way, plate holes 19 and 20 are offset from the midline of plate 10. Further, plate holes 19 and 20 can be directly opposite from one another with respect to the midline, such that plate holes 19 and 20 each intersect with a single axis that is perpendicular to the midline.

In one embodiment of system 100, first distal plate hole 14 and fourth plate hole 19 are each specifically configured so that their respective perimeters confine a fastener inserted therein to be disposed only along a single axis that is dictated by the respective geometric perimeter. The insertion axes defined by first distal plate hole 14 and fourth plate hole 19 can be made to be substantially parallel, such that fasteners inserted in each of holes 14, 19 are confined to be substantially parallel to each other upon insertion. Fifth plate hole 20 can be configured the same way with respect to first distal plate hole 14. Another option for ensuring parallel insertion of the fasteners is by using target guide 200, discussed below. In other embodiments, holes 14, 19, 20 can be variable angle locking holes which allow a 30° locking cone, and screws inserted therein can be positioned parallel to the frontal plane.

The perimeter of each of the plate holes described herein, except for distal slot, is circular. In other embodiments, one or more of the plate holes can be a slot (as described below) or can have a different perimeter, such as oval, triangular, slot-shaped, etc.

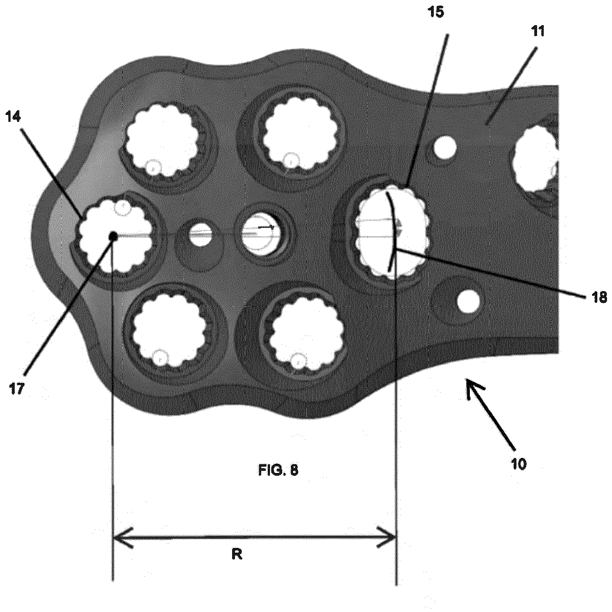

As shown in FIGS. 6 and 7, a perimeter of distal slot 15 is defined by a plurality of spaced apart scalloped regions to facilitate engagement with a head of a fastener. This variable angle locking concept relies on a multi-point form-/friction-fit fixation between plate and screw. This allows for a sufficient interference fit between plate and screw, thus preventing an unintended screw back-out in-vivo. Further disclosure related to the scalloped configuration is found in U.S. Patent. No. 11,039,865, the disclosure of which is incorporated by reference herein. Any or all of the bone and plate holes described herein can also include such a scalloped configuration, as shown in FIG. 8.

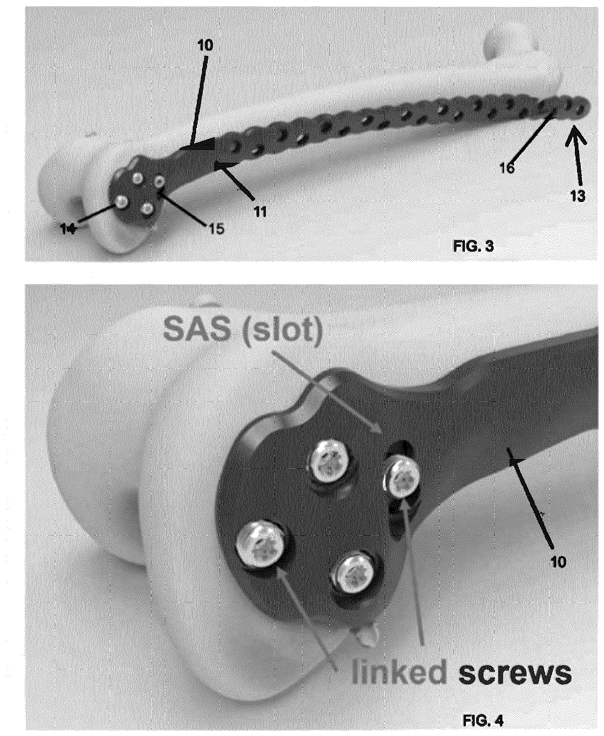

As indicated above, the attempt to utilize two or more distal linkages between a plate and an intramedullary nail could result in a constrained situation for the plate positioning, potentially leading to mal-alignment of the proximal plate area towards the femoral shaft (sagittal plane deviation). As shown in FIGS. 2 and 3, minor mal alignments between distal aspects of nail 70 and plate 10, e.g. caused by the nail entry portal variations, could result in a significant proximal deviation between the bone and plate 10. This kind of mal placement in both anterior of posterior directions has the potential to risk the efficacy of the whole procedure and would be hardly accepted by any surgeon.

In order to substantially reduce the risk of such mal alignment during implantation of plate 10, a slotted locking hole, i.e. distal slot 15, is introduced and acts as a second point of implant interlinking. Distal slot 15 provides the surgeon with the ability to fine tune placement of plate 10 during fixation. After initial insertion and interlinking of the most distal locking screw, for example in distal plate hole 14, this Sagittal Alignment Slot (SAS) 15 still allows for a proper plate-shaft alignment while still ensuring a second interlinking option. Even minimal slot length will allow for potential sufficient position of the proximal plate area.

As shown more clearly in FIG. 8, the configuration of distal slot 15 is designed in connection with the configuration and location of distal plate hole 14. Distal slot 15 has a curved center line 18, and is shown with a slightly exaggerated curve for purposes of this explanation. Center line 18 is a portion of an arc of a circle centered at a center point 17 of first distal plate hole 14. Put another way, distal slot 15 essentially describes an arc which is centered in the distal most hole 14. In this way, distal slot 15 extends along a center line 18 arc of a constant radius from center point 17. A length of the slot along the centerline arc may be 3-25 mm, or 5-20 mm, or 8-15 mm, or can be 10 mm or 12 mm. In other words, a distance R between center point 17 of first distal plate hole 14 to center line 18 is fixed along the entire length of center line 18. This fixed distance R can have a value of between 20-40 mm, or between 20-30 mm, or between 25-30 mm, or can be 26 mm. Based on the configuration and location of distal slot 15 within plate 10, wherever a fastener may be placed within distal slot 15, the head of that fastener within distal slot 15 will be the same distance from the head of a fastener within first distal plate hole 14.

This fixed distance R is also substantially equal to a fixed distance between a center point of first distal nail hole 73 to a center point of second distal nail hole 74. Thus, once a fastener is placed through first distal plate hole 14 and into first distal nail hole 73, a second fastener placed through distal slot 15 will align with second distal nail hole 74 regardless of where within distal slot 15 the second screw is placed.

Additionally, a distance between center point 17 of first distal plate hole 14 to a center point of proximal plate hole 16 is substantially equal to a distance between a center point first distal nail hole 73 to a center point of the proximal nail hole 75, which permits securing the respective proximal and distal ends of plate 10 and nail 70 together. While this permits securing plate 10 to nail 70 at the proximal end, in other embodiments, it does not require the overall lengths of plate 10 and 70 to be identical; they can be the same or different.

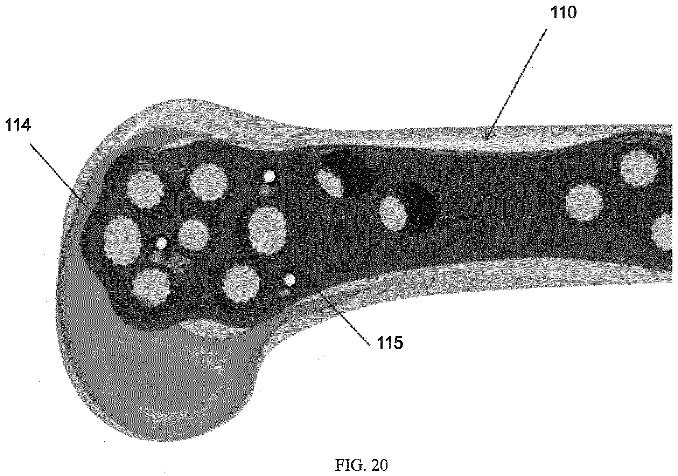

Further embodiments of bone plates 110 and 1110 for use in fracture fixation system 100 are shown in FIGS. 20, 21A, 21B, 22A, and 22B. Plates 110 and 1110 are similar in nature to plate 10, with like elements being similarly numbered. Second distal slots in these plates add more positioning options with regard to degrees of freedom in the anterior-posterior direction. This is particularly useful for easier positioning of the respective plate on the bone while retaining the linking options. The presence of multiple slots increases the flexibility of the plate placement (relative to the nail while still allowing interlinking of both implants) in the anterior-posterior direction. In the plates, the slots have non-circular perimeters and are oriented across the plate, i.e. to extend substantially perpendicular to a midline extending between ends of the bone plate.

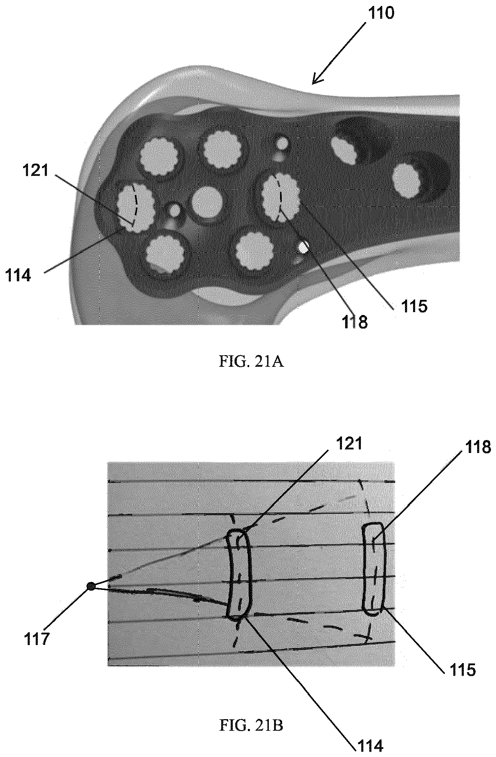

Plate 110, shown in FIGS. 20 and 21A, includes distal slot 115 and the plate holes in the same configuration as plate 10, though replacing first distal plate hole 14 with a second slot 114. That is, aside from slots 114 and 115, the other plate holes have circular perimeters. Distal slot 114 acts as another point of implant interlinking. Other configurations of the perimeters of the non-slot plate holes are also possible.

The configuration of distal slot 115 is the same as discussed above in connection with slot 15, such that it has a curved center line 118. Slot 114 also has a curved center line 121 that is a portion of an arc of a circle. Both slots center line 118 and center line 121 are centered at a center point 117 that does not necessarily coincide with any point or feature on plate 110. In fact, center point 117 is located outside of plate 110. Accordingly, both distal slots 114 and 115 extend along center line arcs of respective constant radii from center point 117. The arrangement of slots 114 and 115 and center point 117 are shown in FIG. 21B.

The distance between two respective points along center lines 118 and 121 includes several combinations for which the distance is substantially equal to a fixed distance between a center point of first distal nail hole 73 to a center point of second distal nail hole 74. Thus, once a fastener is placed through distal slot 114 and into first distal nail hole 73, a second fastener placed through distal slot 115 will align with second distal nail hole 74, while allowing for some flexibility for the exact angling of plate 110 so that it can be properly aligned along the bone with respect to nail 70. The location of distal slots 114 and 115 also facilitates insertion of a fastener in a proximal plate hole, as described above. Using a plate with two curved slots such as plate 110 allows essentially both translations and rotation of plate 110 relative to nail 70.

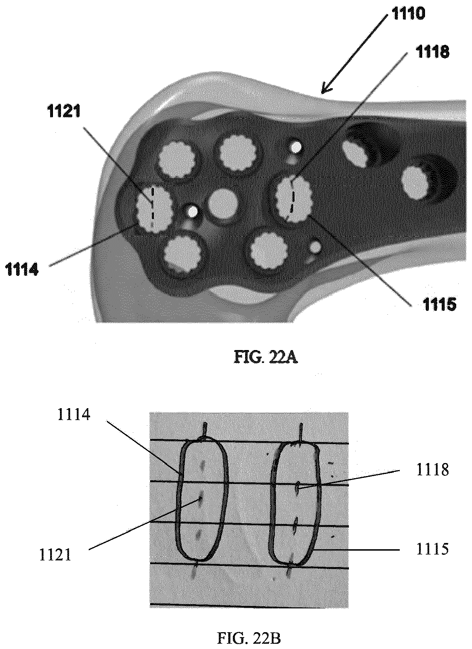

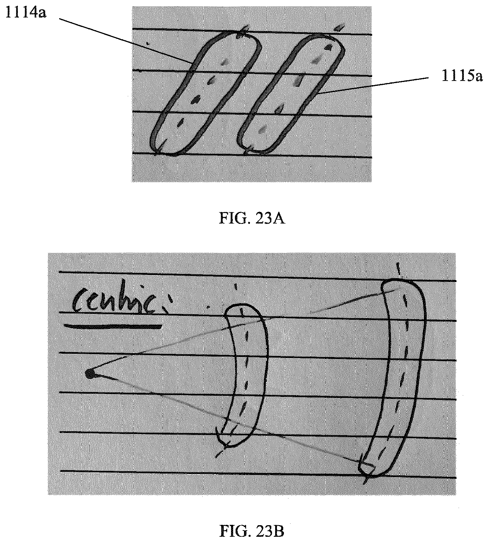

Plate 1110 is shown in FIG. 22A and is similar in nature to plate 110, although both first and second slots 1114, 1115 extend along parallel straight centerlines 1121, 1118, respectively. The arrangement of slot 1114 and slot 1115 is also shown schematically in FIG. 22B. This permits manipulation of plate 1110 before screws within slots 1114, 1115 are tightened, such that plate 1110 can be moved generally anteriorly or posteriorly on the bone and generally in a direction parallel to the axes of centerlines 1121 and. 1118. In another version of the plate shown in FIG. 23A, slots 1114a, 1115a are also parallel with parallel centerlines, but are both angled with respect to a centerline of the plate. This is different from plate 1110 in which centerlines 1121, 1118 are both substantially perpendicular to the centerline of the plate.

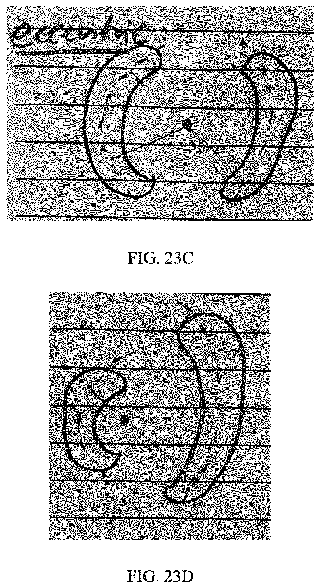

In other versions of distal slots, the curvatures of the slots can be about the same center point to one side of both slots (FIG. 23B), about the same center point between both slots so that the slots are symmetrical about the center point (FIG. 23C), or about the same center point between both slots that is closer to one slot than the other (FIG. 23D). The curvature of the centerline can be toward the proximal end of the plate about a point between the slot(s) and the proximal end of the plate (such as in FIG. 23B). In other versions, a plate can have two distal slots in which the respective centerlines are curved to be concave toward each other to be centered on the same point between the slots (such as FIGS. 23C and 23D).

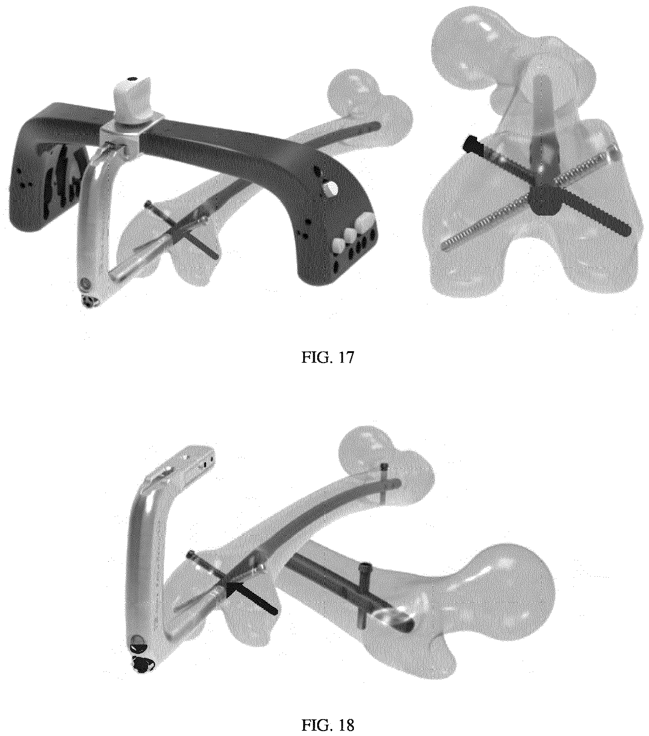

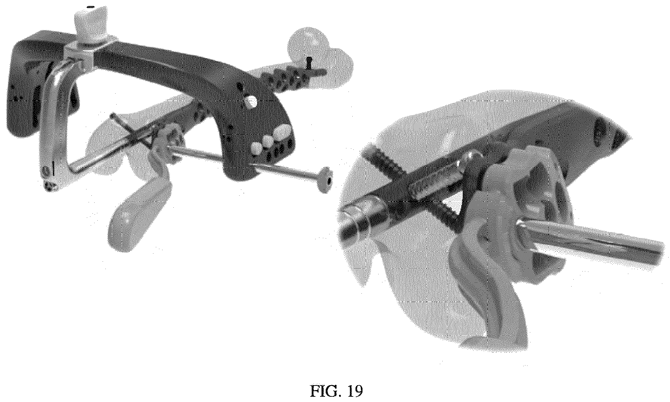

A method of using fracture fixation system 100 is described below, generally with respect to plate 10. It will be understood that much of the description applies to plates 110 and 1110 as well even though plate 10 is referenced, and additional clarification is provided where appropriate. The method includes first inserting nail 70 into a canal of a bone such as a tibia, a humerus, or a femur, and inserting medial and or lateral screws into nail 70 using a targeting device attached to nail 70 to establish distal locking, as shown in FIG. 17. Subsequent to distal nail locking, proximal locking of nail 70 is performed by using one or two locking screws in anterior-posterior direction, as shown in FIG. 18. Once the nail locking is completed, an appropriately sized plate is chosen, such as plate 10, 110, or 1110. As shown, plate 10 is mounted to an inserter/targeting handle and subcutaneously inserted or placed against a surface of the bone, such that distal end 12 of plate 10 is adjacent to, i.e. at the same end of the long bone as, distal end 71 of nail 70. The correct relative position between nail 70 and plate 10 can be obtained by using the distal targeting arm connected with nail 70 and the corresponding sleeve system within the most distal locking hole to push (preliminarily fix) the plate-inserter assembly towards the lateral cortex of the femur, as shown in FIG. 19. In one embodiment, nail 70 is a retrograde femoral nail that is inserted into an entry location at a distal end of a femur.

Next, plate 10 and nail 70 are provisionally aligned and fixed. This is done by aligning first distal plate hole 14 with first distal nail hole 73, as shown in FIG. 19, and then inserting an alignment instrument through first distal plate hole 14, into the bone, and through first distal nail hole 73 of the implanted nail 70. The alignment instrument can be a k-wire or any similar temporary fixation device. To properly align plate 10 with nail 70 along the entire length of the bone, two other holes of plate 10 and nail 70 are then aligned. This can include aligning proximal plate hole 16 with proximal nail hole 75, or another pair of holes that facilitates the same alignment.

Next, a first distal bore is drilled in the bone through first distal plate hole 14 and through first distal nail hole 73, for example by using target guide 200 discussed below. This can be done using a targeting device attached to nail 70, in a similar manner to the way in which the locking screws are inserted into nail 70, described above. This can occur over the k-wire using a cannulated drill, or else the k-wire can be removed for drilling to occur. With the first distal bore drilled, a first fastener 52 is inserted through first distal plate hole 14 and into the first distal bore and first distal nail hole 73 to couple nail 70 and plate 10 at their respective distal ends. When plate 110 or plate 1110 is utilized, this step involves inserting first fastener 52 through slot 114 or slot 1114, respectively, without tightening first fastener 52 since the position of first fastener 52 within the respective slot 114, 1114 may change. The same is true for the slot arrangements shown in FIGS. 23A-D. Maintaining the overall alignment between plate 10 and nail 70, a second distal bore is then drilled in the bone through distal slot 15 and second distal nail hole 74. A second fastener 56 is then inserted through distal slot 15, into this second distal bore, and into second distal nail hole 74. With respect to any of the described plates, and particularly for plate 110 or plate 1110, second fastener 56 is not yet tightened at this point.

When utilizing plate 10, the provisional fixation of the method is done using first distal plate hole 14 since it is the hole by which the configuration and geometry of distal slot 15 are defined. In this way, a proper placement of plate 10 on the bone, i.e. not too anterior or posterior, can be performed while ensuring that it remains possible to realize a second distal link, which is facilitated by the geometry of distal slot 15. That is, even if the initial placement of plate 10 against bone and the steps of initially securing the distalmost end of plate 10 to nail 70 with a single fastener is not in the most optimal anatomic location, further alignment of the proximal end of plate 10 can be achieved while maintaining and ensuring the ability for secondary distal fixation through distal slot 15. The configuration of distal slot along curved center line 18 makes this possible, since slight changes in the proximal location of plate 10 with respect to the bone allow a fastener to be inserted into distal slot 15, albeit at one of a plurality of locations along distal slot 15 that are each the same fixed distance R to first distal plate hole 14, and thereafter into the second distal bore and second distal nail hole 74.

A similar functionality is provided by plate 110 and plate 1110 and the slot arrangements shown in FIGS. 23A-D, for which proper placement is allowed by manipulating the respective plate into an optimal location where first and second fasteners 52, 56 can each be moved in their respective slots. Plate 110 can be easily pivoted about point 117, so that proper alignment can be achieved. Plate 1110 can be manipulated so that plate is moved posteriorly and anteriorly along the bone. The slot arrangement in FIG. 23A allows translation of the plate along a direction parallel to the slot centerlines, while the slot arrangements in FIGS. 23B-D allow rotation of the plate about the center point of the curved slot centerlines. In this regard, during the method, the steps of inserting first fastener 52 and second fastener 56 without tightening, the plates can be moved with respect to the bone in an anterior-posterior direction, i.e. in a direction that is generally perpendicular to a longitudinal axis of the plate, or by being rotated with respect to the bone. This movement can be a pivoting about the fastener disposed at the proximal end of the plate.

Additional screws can be used for fixation of distal end 12 of plate 10 to the underlying bone. A fourth bore is drilled through plate hole 19 and a fifth bore is drilled through plate hole 20. Importantly, both bores drilled through holes 19 and 20 are performed to avoid alignment with a hole in nail 70, and more specifically to avoid contact with nail 70 altogether. As shown in FIG. 5, a fastener 58 is inserted into fourth plate hole 19 and a fastener 59 is inserted into fifth plate hole 20. As indicated above, the insertion axes defined by fourth plate hole 19 and fifth plate hole 20 can be made to be substantially parallel, which dictates that fasteners 58 and 59 inserted therein are confined to be substantially parallel to each other upon insertion. As such, both fasteners 58 and 59 are inserted to avoid contact with nail 70, and the surgeon can have high confidence that this will be true due to the strict configuration of fourth plate hole 19 and fifth plate hole 20. In various embodiments of the method, either or both of fasteners 58 and 59 can be utilized as needed for added fixation of plate 10 to the bone. Subsequent to distal fixation, the plate can be locked along the femoral shaft for optimized construct strength and stability. At this point in the method with the plate properly located with respect to the bone, all fasteners can be tightened into their final implanted locations. This step of tightening can occur earlier during the procedure as needed as well.

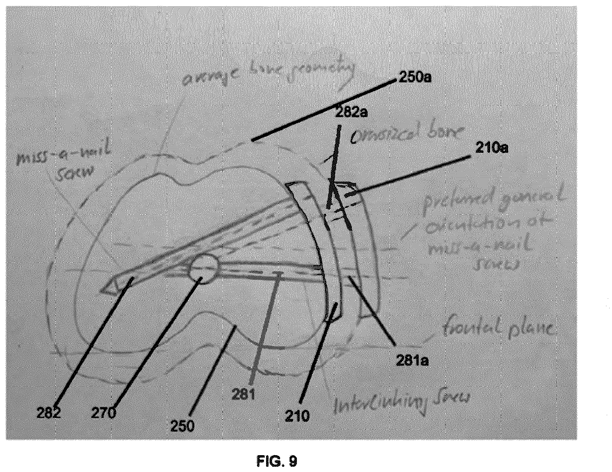

A primary reason for the configuration of fourth plate hole 19 and fifth plate hole 20 is demonstrated by FIG. 9, which shows a cross-section of a distal end of a femur. Considering space limitations for adequate screw placement within the affected bone and the availability of variable angle locking systems, allowing a higher flexibility in screw trajectories, surgeons are facing an increased risk of unintended collision between screw and intramedullary nail. This might lead to a drastically reduced fatigue life of the affected implants. A solution to avoid screw-nail collision might be given by special fixed angle drill sleeves or targeting blocks eliminating the locking variability by serving the zero-degree trajectory of the locking holes only. This technique comes with the disadvantage that the respective locking screws cannot be placed according to surgical preferences (e.g. targeting the area of best bone quality or avoiding collisions with other implants or sensitive anatomical structures).

In FIG. 9, an outer perimeter 250 of an average sized bone is shown along with a bone plate 210 placed on its surface and a bone nail 270 disposed within the intramedullary cavity. Here it is seen that a corresponding first fastener 281 is passed through a hole in plate 210 that is configured to be aligned with a hole in nail 270. A second fastener 282 is passed through another hole in plate 210 that is configured for fixation only with the surrounding bone and not for interaction with nail 270. It is shown that fastener 282 projects near nail 270 but does not contact or interfere with nail 270 at all. However, an outer perimeter 250a of an oversized sized bone is also shown overlaid in FIG. 9. A bone plate 210a that is identical to bone plate 210 is placed on its surface. A corresponding first fastener 281a is passed through the same hole in plate 210a that is configured to be aligned with a hole in nail 270. A second fastener 282a is passed through the same other hole in plate 210a that is configured for fixation only with the surrounding bone and not for interaction with nail 270. This highlights a critical problem with use of a similar bone plate on differently sized bones, in that fastener 282a is now aimed to directly contact nail 270, which is not intended or desired during implantation of plate 281a. Thus, use of a single bone plate is not always possible given the differences in size and configuration of different bones.

This problem is addressed by plate 10 (and the other plates disclosed herein) of the present invention, in which the insertion axes defined by fourth plate hole 19 and first distal plate hole 14, along with the axis defined by fifth plate hole 20 in some embodiments, are made to be substantially parallel. Thus, no matter the distance from the implanted nail 70 to the outer perimeter of the bone, the surgeon can proceed with confidence that the configuration of the holes will prevent unintended contact between nail 70 and the fasteners that are intended to be used for fixation only between plate 10 and the bone, i.e. fasteners 52, 58, and 59. In other words, those screws will be aligned to provide a user with a standard parallel screw trajectory to prevent nail damage. For oversized bone dimensions, in an angled trajectory there is the danger of touching the nail body, while with a parallel approach this is prevented.

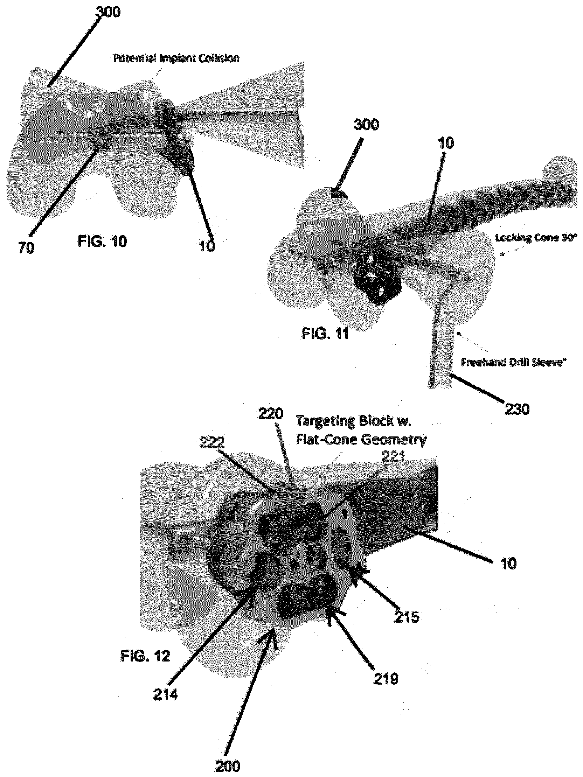

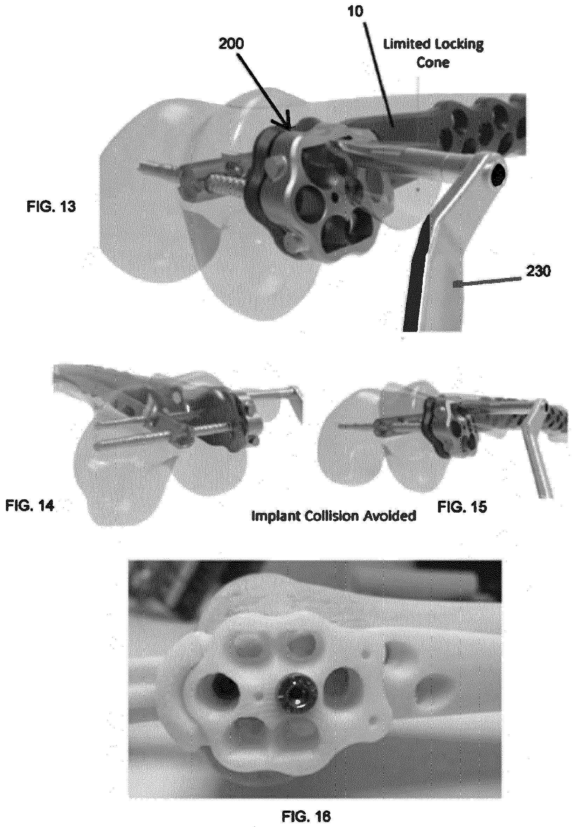

In other embodiments, the various plate holes of plate 10 (and the other plates disclosed herein) may not be configured to confine insertion of a fastener along a particular axis, and may instead provide for variably angle insertion of fasteners therein. In that case, additional assistance in avoiding contact between a fastener and nail 70 is provided by a target guide 200 shown in FIGS. 10-16, which provides a solution for maintaining the variability within the angled locking trajectory of the plate hole the while excluding certain regions of the cone of insertion.

FIG. 10 displays the risk of implant collisions within a nail-plate-combination construct of the distal femur. By utilizing a 30-degree locking cone of the locking mechanism, an interference/collision with nail 70 can be avoided by a cautious and x-ray supported screw insertion process only. That is, the cone 300 defines the angles (in this case, up to 30 degrees from normal to the plate hole) within the particular plate hole within which a fastener can be inserted. It is seen that a lower portion of cone 300 intersects nail 70 at a location denoted "Potential Implant Collision," demonstrating that contact between an inserted fastener and nail 70 would occur in such instances.

Utilizing a freehand drill sleeve 230 in combination with target guide 200, the locking cone 300 can be constrained selectively to avoid screw-nail collisions while maintaining generally flexibility for fastener insertion for all other directions. Beside safety aspects, such guided techniques can increase speed and decrease x-ray exposure within the respective procedure.

Target guide 200 is removably connected to distal end 12 of plate 10, such as by snap fit or by connection with a set screw. While this description relates to plate 10, it applies equally to the other plates disclosed herein. A guide hole 214 aligns with first distal plate hole 14 (or a slot at this location if plate 110 or 1110 or any of the other slot configurations is used), and a guide slot 215 aligns with distal slot 15. For plate holes 19 and 20, guide 200 includes flat guide holes 219 and 220, respectively. Flat guide hole 220, for example, has a conical surface 221 intersected by a planar surface 222 at one side. Since an outer diameter of drill sleeve 230 is lesser than an inner diameter of flat guide hole 220, drill sleeve 230 can be inserted into flat guide hole 220 until a distal end of drill sleeve 230 is engaged with or secured to plate hole 20. Once this is done, drill sleeve 230 can be pivoted within plate hole 20 according to surgeon preference to direct the drill and eventual fastener into a location of the surgeon's choice within the bone. However, planar surface 222 prevents drill sleeve 230 from pivoting upward (with reference to FIG. 12, such that the drill and eventual fastener are limited from being directed toward nail 70, thereby preventing contact between the fastener and nail 70. While guide hole 214 and guide slot 215 do not contain planar walls since they are intended to guide a fastener into a corresponding hole in nail 70, any other guide hole in guide 200, such as flat guide holes 219 and 220, can be specifically designed based on the dimensions of plate 10 with which guide 200 is designed for use so that the flat surfaces of the guide holes create limited locking cones to avoid fastener-nail contact. This preserves the autonomy of the surgeon while providing a failsafe to avoid unintended contact between a fastener and nail 70.

As an additional step in the surgical method, proximal fixation of plate 10 can be performed, though it is not required. An alignment instrument is inserted through proximal plate hole 16, into the bone, and through proximal nail hole 75 in order to align and fix proximal end 13 of plate 10. A proximal bore is then drilled in the bone either over or after removal of the k-wire, with the bore extending through proximal plate hole 16 and proximal nail hole 75. Then a third fastener is inserted through proximal plate hole 16 and into the proximal bore and proximal nail hole 75.

The drilling steps described above can be performed in accordance with industry standards and techniques, and can include using a targeting arm attached to nail 70 and/or a drill sleeve for guidance and aiming of the drill. The alignment and drilling steps may utilize intraoperative fluoroscopy in order to properly place and align the components of system within the bone.

Each of the fasteners described herein can be a screw with a threaded or non-threaded head, or an unthreaded peg. In one embodiment, screws with threaded heads are inserted into each of the holes and distal slot of plate 10 during the method. In another embodiment, screws with threaded heads are inserted into each of the holes of plate 10, while an unthreaded peg is inserted into distal slot 15. Any combination of the same or different types of fasteners can be employed in a particular procedure based on need and/or surgeon preference.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A fracture fixation system, comprising:
a bone nail configured to be inserted into a canal of a bone; and
a bone plate configured to abut a surface of the bone;
wherein the bone nail includes first, second, and third nail holes, the second nail hole located between the first and third nail holes, and
wherein the bone plate includes first and third plate holes and a slot located between the first and third plate holes.

2. The fracture fixation system of claim 1, wherein a distance between a center point of the first plate hole to a center line of the slot is substantially equal to a distance between a center point of the first nail hole to a center point of the second nail hole.

3. The fracture fixation system of any of claims 1-2, wherein the slot of the bone plate has a center line extending along an arc of a constant radius from a center point of the first plate hole.

4. The fracture fixation system of claim 3, wherein a length of the slot along the centerline is 3-25 mm.

5. The fracture fixation system of any of claims 1-4, wherein the slot extends substantially perpendicular to a midline of the bone plate extending between ends of the bone plate.

6. The fracture fixation system of any of claims 1-2, wherein the slot of the bone plate has a center line extending along an arc of a constant radius, wherein the first plate hole is a slot having a center line extending along an arc of a constant radius.

7. The fracture fixation system of claim 6, wherein the center points of the arcs are the same.

8. The fracture fixation system of claim 6, wherein the center points of the arcs are different.

9. The fracture fixation system of claim 6, wherein the slots extend substantially perpendicular to a midline of the bone plate extending between ends of the bone plate.

10. The fracture fixation system of any of claims 1-2, wherein the slot of the bone plate has a center line extending along an axis substantially perpendicular to a midline of the bone plate extending between ends of the bone plate.

11. The fracture fixation system of any of claims 1-10, wherein a distance between a center point of the first plate hole to a center point of the third plate hole is substantially equal to a distance between a center point of the first nail hole to a center point of the third nail hole.

12. The fracture fixation system of any of claims 1-11, wherein a perimeter of the slot is scalloped to engage a head of a screw and to prevent backout.

13. The fracture fixation system of any of claims 1-5 and 10-12, wherein each of the first plate hole, the third plate hole, the first nail hole, the second nail hole, and the third nail hole has a circular perimeter, and the slot has a non-circular perimeter.

14. The fracture fixation system of any of claims 1-12, wherein each of the third plate hole, the first nail hole, the second nail hole, and the third nail hole has a circular perimeter, wherein the slot has a non-circular perimeter, and wherein the first plate hole is a slot having a non-circular perimeter.

15. The fracture fixation system of any of claims 1-14, wherein the bone plate further defines a fourth plate hole located between a first side edge of the bone plate and a midline of the bone plate extending between ends of the bone plate, and wherein the first plate hole defines a perimeter confining a fastener to insertion along a single axis, and the fourth plate hole defines a perimeter confining a fastener to insertion along an axis parallel to the single axis.
